# EUROPEAN PATENT SPECIFICATION

(11) **EP 2 116 257 B1**
(45) Date of publication and mention of the grant of the patent: **31.10.2012**
(21) Application number: 09166586.9
(22) Date of filing: 09.08.2001
(51) Int. Cl.: A61K 39/39, A61P 37/04

(54) **Parenteral vaccine formulations and uses thereof**
Parenterale Impfstoff-Formulierungen und deren Verwendung
Formulations parenterales de vaccin et applications de celles-ci

(30) Priority: 09.08.2000 DK 200001194; 09.08.2000 US 224037 P
(43) Date of publication of application: 11.11.2009
(62) Divisional of application: 01957778.2
(73) Proprietor: Alk-Abelló A/S, 2970 Hørsholm (DK)
(72) Inventor: Soni, Nanna Kristensen, 1366 Copenhagen K (DK); Rahbek, Janne Uldal, 2840 Holte (DK); Aasmul-Olsen, Stig, 2942 Skodsborg (DK); Lund, Lise, 3480 Fredensborg (DK)
(74) Representative: Olsen, Henrik Bagger

(56) References cited:
- EP-A- 0 445 710
- WO-A-00/45847
- SINGH M; O'HAGAN D: "ADVANCES IN VACCINE ADJUVANTS" NATURE BIOTECHNOLOGY, NATURE PUBLISHING GROUP, NEW YORK, NY, US, vol. 17, no. 11, 1 November 1999 (1999-11-01), pages 1075-1081, XP000999107 ISSN: 1087-0156
- S. COSTAGLIOLA ET AL.: "Recombinant thyrotropin receptor and the induction of autoimmune thyroid disease in BALB/c mice: a new animal model." ENDOCRINOLOGY, vol. 135, no. 5, 1994, pages 2150-2159, XP001009706 Philadelphia, US
- LEVINE L; STONE J L; WYMAN L: "FACTORS AFFECTING THE EFFICIENCY OF THE ALUMINUM ADJUVANT IN DIPHTHERIA AND TETANUS TOXOIDS" JOURNAL OF IMMUNOLOGY, AMERICAN ASSOCIATION OF IMMUNOLOGISTS, US, vol. 75, no. 4, 1 October 1955 (1955-10-01), pages 301-307, XP008064584 ISSN: 0022-1767
- STAS' N F; KONOVALOVA Z S; MARCHENKO N A: "MODIFICATION OF ALUMINUM HYDROXIDE USED AS AN ADJUVANT" KHIMIKO-FARMATSEVTICHESKII ZHURNAL, MOSCOW, RU, vol. 65/66, no. 24, 1 January 1990 (1990-01-01), page 7, XP008064498 ISSN: 0023-1134 & DATABASE BIOSIS BIOSCIENCES INFORMATION SERVICE, PHILADELPHIA, PA, US; 1990, STAS' N F ET AL: "MODIFICATION OF ALUMINUM HYDROXIDE USED AS AN ADJUVANT" XP002169722
- WEDRYCHOWICZ H; BEZUBIK B: "Influence of adjuvants on immunity in rabbits vaccinated with infective larval somatic proteins of Trichostrongylus colubriformis" VETERINARY PARASITOLOGY, ELSEVIER SCIENCE, AMSTERDAM, NL, vol. 37, no. 3-4, 1 November 1990 (1990-11-01), pages 273-284, XP023712333 ISSN: 0304-4017 [retrieved on 1990-11-01] & DATABASE BIOSIS BIOSCIENCES INFORMATION SERVICE, PHILADELPHIA, PA, US; 1990, WEDRYCHOWICZ H ET AL: "INFLUENCE OF ADJUVANTS ON IMMUNITY IN RABBITS VACCINATED WITH INFECTIVE LARVAL SOMATIC PROTEINS OF TRICHOSTRONGYLUS-COLUBRIFORMIS" XP002169721
- J.Y. LEE ET AL.: "Beryllium, an adjuvant that promotes gamma interferon production." INFECTION AND IMMUNITY, vol. 68, no. 7, July 2000 (2000-07), pages 4032-4039, XP002169720 WASHINGTON, US

## Description

The present invention concerns the field of parenteral vaccine formulations and adjuvant compositions comprising certain salts as adjuvants. Such novel parenteral vaccine formulations are used for generating an immune response in a subject, including a vertebrate such as a human, following administration of the vaccine formulation. The invention further relates to the use of these salts as adjuvants in parenteral vaccine formulations and adjuvant compositions, and to vaccine adjuvants comprising such salts.

### BACKGROUND OF THE INVENTION

Immunological adjuvants are substances that, when administered together with an antigen, have the capacity to augment the immune response to the antigen. When used as a component in a vaccine formulation, the adjuvant improves the immunogenicity of the vaccine in the sense that it will enhance the immune response of the vaccinated subject, thereby reducing the amount of the vaccine component, or reducing the number of administrations needed to induce the desired immune response.

The induced effector immune response may either be of a humoral nature, i.e. an antibody response, or of a cellular nature, i.e. a cytotoxic T-cell response, or the effector response may be a mixture of both. Both cellular and humoral immune responses require help from T helper lymphocytes. Adjuvants that cause inflammation or induce pro-inflammatory cytokines will induce a Type-1 T helper response (Th₁) involving production of IL-12, IL-2 and INFγ. These cytokines support induction of cytotoxic T-lymphocyte (CTL) responses, neutrophil inflammation and Th₁ antibody responses, such as IgG1 and IgG3. Non-inflammatory adjuvants are more likely to induce a Type-2 helper response (Th₂) involving production of the cytokines IL-4, IL-5 and IL-10. These cytokines can down-regulate Th₁ responses, and promote induction of Th₂ antibodies such as IgE and IgG4 as well as some cellular responses such as eosiniphilia.

Although adjuvants have been applied in the field of immunology and vaccine technology for many years, the underlying mechanisms of action are not completely understood. This has certainly complicated targeted research for identifying new adjuvant candidates.

Several substances have been or are currently being investigated for their adjuvant properties. A few examples are aluminium salts, PLG (polylactide co-glycolide), oil in water emulsions such as MF59 (a squalene in water emulsion), Quil A, Qs-21 and ISCOMs. However, many of the tested adjuvants have several drawbacks, including ineffectiveness with some antigens, contact hypersensitivity, subcutaneous nodules, and granulomatous inflammation. Others still await critical evaluation in clinical trials.

Singh, M., and O'Hagan D., provides an overview on vaccine adjuvants and discloses aluminium hydroxide and calcium phosphate as adjuvants and indicate that they are administered by injection.

Costagliola et al., discloses vaccine formulations for i.p. administration, comprising an immunogenic substance (MBP-ECD) and magnesium hydroxide and aluminium hydroxide as adjuvants.

Thus, there is still a strong need for improved or safer adjuvants which can be used in human vaccines.

### SUMMARY OF THE INVENTION

It has surprisingly been found that certain salts are particularly suited as adjuvants in vaccine formulations for parenteral administration. Such salts are further particularly suited as components of adjuvant compositions.

Thus, the present invention concerns parenteral vaccine formulations comprising at least one immunogenic substance, and as an adjuvant titanium dioxide.

In further aspects, the present invention concerns the use of titanium dioxide as an adjuvant as components of vaccine formulations for parenteral administration.

The present invention also enables vaccination or treatment by administration of the vaccine formulation according to the invention.

Furthermore, the present invention relates to a process for preparing the adjuvant compositions and parenteral vaccine formulations of the invention.

### BRIEF DESCRIPTION OF THE FIGURES

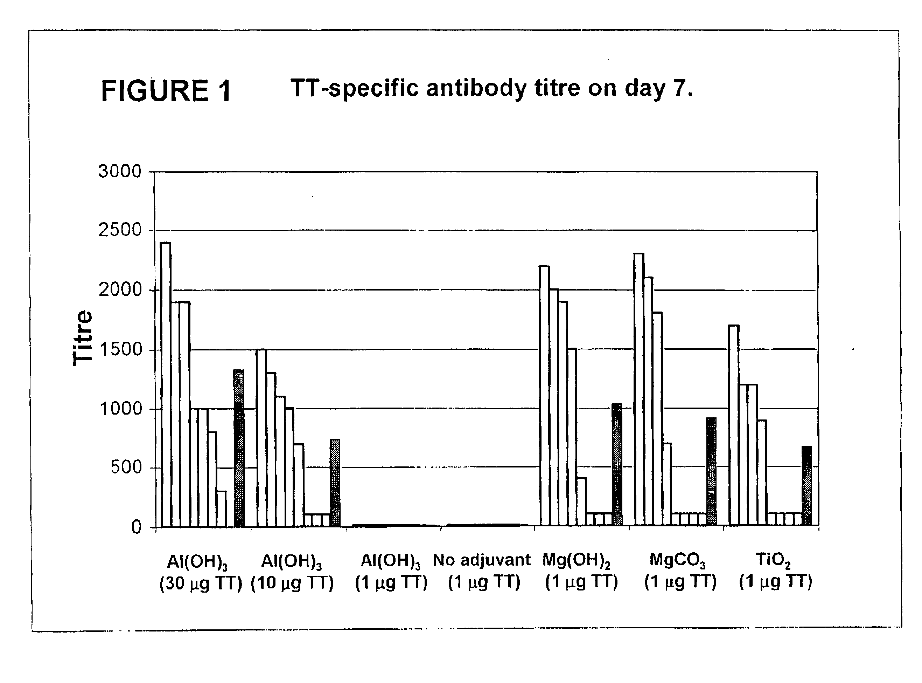

Figure 1 shows the results obtained in Example 1 with parenteral vaccine formulations according to the invention. As adjuvant is used magnesium hydroxide, magnesium carbonate hydroxide pentahydrate (in short MgCO₃ ), or titanium dioxide. As immunogenic substance is used *Tetanus toxoid.* The results are compared to vaccine formulations either containing aluminium hydroxide as adjuvant, or containing no adjuvant, and as immunogenic substance *Tetanus toxoid.*

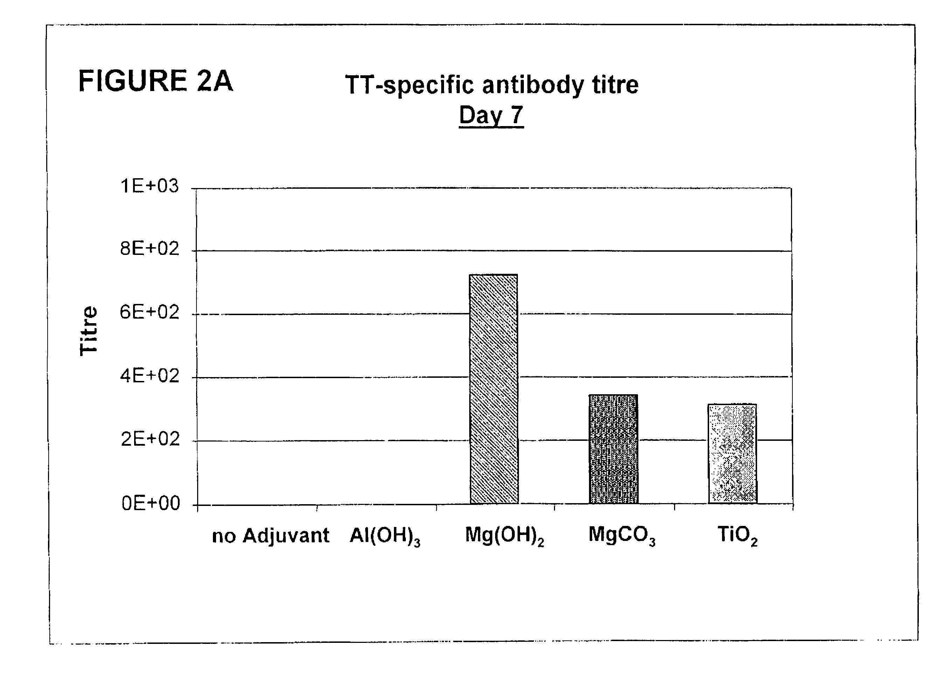

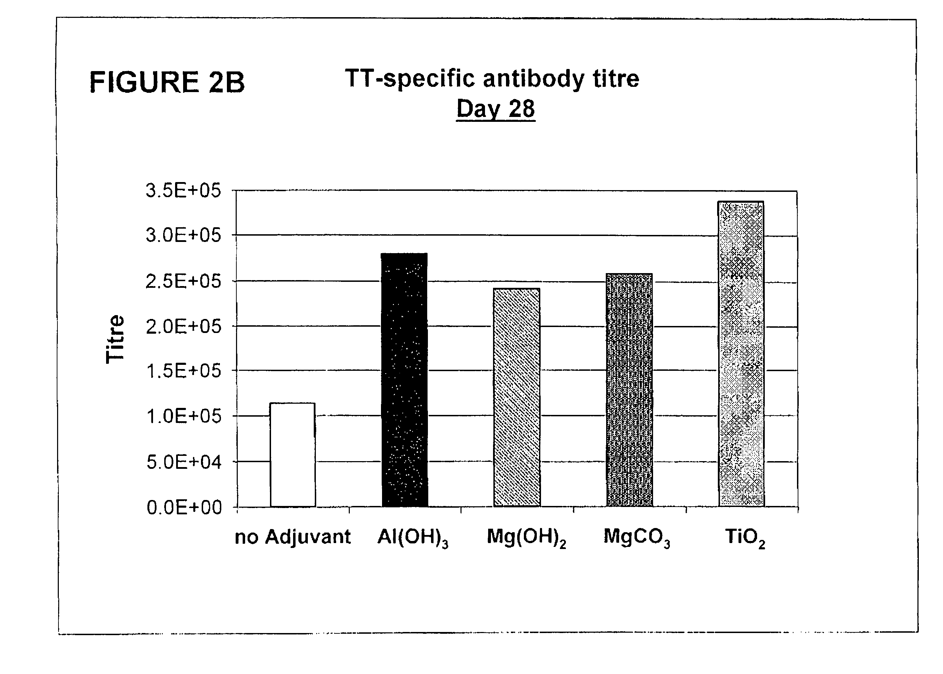

Figures 2A and 2B show the results obtained in Example 2 with parenteral vaccine formulations of the invention. As adjuvant is used magnesium hydroxide, magnesium carbonate hydroxide pentahydrate (in short MgC0₃ ), or titanium dioxide. As immunogenic substance is used *Tetanus toxoid.* The results are compared to vaccine formulations either containing aluminium hydroxide as adjuvant, or containing no adjuvant, and as immunogenic substance *Tetanus toxoid.*

### DETAILED DESCRIPTION OF THE INVENTION

As mentioned above, the mechanisms of action of adjuvants are not completely understood. There are, however, some general theoretical principles by which an adjuvant may exert its effect, namely
(1) the adjuvant may create a depot of the antigen resulting in a prolonged slow release over time, thereby reducing the need for booster vaccinations,
(2) the adjuvant may enhance antigen uptake by antigen presenting cells (APCs) allowing the antigen to gain access to intracellular antigen processing pathways, and
(3) the adjuvant may be pro-inflammatory, i.e. attract and activate macrophages, monocytes, and other cells of the immune system, or stimulate cytokine production.

Furthermore, several other factors relating to adjuvanicity are believed to promote the immunogenicity of antigens. These include
(1) rendering antigens particulate, e.g. aluminium salts,
(2) polymers or polymerisation of antigens,
(3) slow antigen release, e.g. emulsions or micro-encapsulation,
(4) bacteria and bacterial products, e.g. CFA,
(5) other chemical adjuvants, e.g. poly-I:C, dextran sulphate and inulin,
(6) cytokines, and
(7) antigen targeting to APC.

While a number of adjuvants (e.g. aluminium salts, PLG, ISCOMs, MF59, MPL, IFA (Freund's incomplete adjuvant), CFA (Freund's complete adjuvant), Quil A, and Qs-21) have been used in experimental and veterinary medicine, aluminium salts (aluminium hydroxide and aluminium phosphate) are currently the only adjuvants used routinely in humans.

Aluminium based vaccines are most commonly manufactured by mixing pre-formed aluminium phosphate or hydroxide gels with the antigen, resulting in adsorption of the antigen to the gel. Aluminium salts are believed to exert their effect as adjuvants by several mechanisms. It is believed that aluminium salts create a depot of antigen at the site of injection, and attract various cells of the immune system, thus creating a local inflammatory environment. Aluminium salts are most likely taken up by antigen presenting cells. Particles of a size of or less than 10 µm are effectively taken up by antigen presenting cells. The particle size of aluminium gels is 0.5-10 µm, making this plausible (see ref. 6).

Although aluminium salts are relatively cheap and easy to manufacture and, in addition, have an excellent track record of safety and adjuvanticity, there are certain problems connected with the use of aluminium compounds. These include local reactions such as erythema, contact hypersensitivity, subcutaneous nodules, and granulomatous inflammation, augmentation of specific and total IgE antibody responses (an undesired antibody type in certain pathogenic conditions such as allergies) in both experimental animals and in humans. In addition, aluminium salts have been reported to be ineffective as adjuvants with some antigens, cf. Gupta et al. (ref. 1). A disturbing observation has further been reported. It has been suggested in some studies that aluminium can cause experimental degenerative disorders of the central nervous system, cf. Rao et al. (ref. 2). Although there, as yet, is no clinical evidence linking the use of aluminium-containing vaccines to the development of human neuro-degenerative disorders such as Alzheimer's disease, a connection has been postulated and widely debated, cf. Savory et al. (ref. 3). This has generated some anxiety concerning the continuous use of aluminium-containing adjuvants in humans, and the search for alternative, aluminium free adjuvants has been called for.

Also the use of magnesium hydroxide in combination with aluminium hydroxide has been studied. While magnesium hydroxide alone has a low adsorbent activity, it may enhance the activity of aluminium hydroxide as an adjuvant, cf. Stas et al (ref. 8).

Beryllium being the lightest metal with an atomic weight of 4 and a molecular weight of 9 has been known to act as an antigen and further it has been shown to act as an adjuvant promoting the production of IFN-γ and enhancing the properties of IL-12 in a study on Leishmania-susceptible mice, which are immunized with soluble leishmanial antigen. The ability of beryllium to synergize with IL-12 may be an important factor in the development of adjuvants. Accordingly, beryllium has been suggested potential for developement as a vaccine adjuvant, cf. Lee et al (ref. 7).

Thus, many adjuvant candidates have been suggested. Many of these promising new adjuvant candidates have, however, failed to pass the developmental stage, because they have been shown to lack one or several of the characteristics desired for adjuvants, when tested in pre-clinical trials, cf. Newman (ref. 4). Many adjuvant candidates have displayed unacceptable levels of toxicity, thus limiting the applicability to very serious conditions, such as chronic viral infection, cancer or HIV therapy, where the use of adjuvants inducing higher levels of local or systemic side effects may be more acceptable. Some of the candidates have proven too costly to manufacture, or the manufacturing and purification process is too complicated to be feasible. Furthermore, a good adjuvant for human use should display physicochemical stability for longer periods, preferably a year, and some adjuvant candidates have proven to have a limited shelf life.

PLG polymers were initially developed for use as biodegradable surgical sutures, and depot-formulation of various hormones. The characteristics of PLG (biodegradability, documented safety in humans, and relatively easy manufacturing) made it an obvious vaccine delivery candidate. The system is based on encapsulation of the antigen, and, if necessary, other components in 1-100 µm microspheres. When the particle is degraded, the antigen is released. The kinetics of the release can be adjusted by altering the composition of the polymers. Smaller microspheres (≤10 µm) will be taken up by antigen presenting cells, thus targeting the antigen to immunocompentent tissues. The main drawback of the PLG technology is the harsh physical and chemical conditions needed in the manufacturing process, which may render this technology useless with certain labile antigens. Although a good safety profile of PLG has been established, the adjuvanticity of PLG vaccines still awaits critical evaluation in clinical trials, cf. Newman (ref. 4).

Oil in water emulsions such as MF59 (a squalene in water emulsion) are more liquid than water in oil emulsions, and are therefore not intended to form a depot of antigen at the site of injection. Rather, it is believed that the oil in water emulsions exert their adjuvant effect when droplets are taken up by antigen presenting cells, cf. O'Hagan et al. (ref. 5). The emulsion is prepared and subsequently the antigen added, thus making the technology suitable for fragile or labile antigens, or purposes where the retention of the three dimensional structure of the antigen is required, cf. Newman (ref. 4). The potency of MF59 vaccines is reportedly up to 50-fold higher than vaccines delivered as aluminium salt formulations, in a number of animal models, cf. O'Hagan et al. (ref. 5). The MF59 adjuvant reportedly induces an antibody response, rather than a cellular immune response, and is thus better suited for use in vaccines aiming at augmenting or inducing antibody responses, cf. Newman (ref. 4).

Other adjuvant systems such as Quil A, Qs-21 and ISCOMs are based on natural products, namely saponins (sterol and triterpenoid glycosides, derived from the bark of the Quilaja saponiaria tree). Quil A is a crude preparation of more than 20 Quilaja saponins, and is currently used for veterinary vaccine technology. However, owing to the heterogenecity and toxicity of the product, Quil A is not suitable for use in human vaccines. This lead to further purification of the individual saponins in Quil A. Experimental vaccine and toxicology studies led to the identification of the Qs-21 as the saponin best suited for use in human vaccines, cf. Newman (ref. 4). Both Quil A and Qs-21 can elicit cellular immune responses as well as antigen responses in experimental animals. Qs-21 is currently being tested in clinical trials. ISCOMs (immunostimulatory complexes) are spherical, hollow particles composed of cholesterol, saponin and phospholipid. Vaccine studies in animals have shown that lower doses of saponins are required to induce similar immune responses, when ISCOMs formulations are used. However, ISCOMs are relatively complicated to manufacture, and the advantage over Qs-21 is still debated, cf. Newman (ref. 4).

As evident from the above, there is a need for suitable adjuvants, in particular such suited for human use. There are several key features which an ideal adjuvant candidate should most preferably display, namely
(1) Safety. The adjuvant should preferably be sufficient non-toxic and biodegradable, and preferably induce minimal local and systemic reactions.
(2) Potency. The adjuvant should preferably be able to reduce the amount of antigen and/or number of applications needed in order to induce a long-lasting immune response (be it an antibody or cellular response).
(3) Stability. The adjuvant should preferably be stable for longer periods, preferably for more than a year, at 4°C or room temperature. Also, the manufacturing process should not have any detrimental effect on the antigen.
(4) Manufacturing and costs. The adjuvant should preferably be easy and cheap to manufacture in order not to increase the manufacturing costs of the vaccine tremendously.

The present invention provides adjuvants which fulfil some or all of the above criteria. It has surprisingly been found that certain salts are capable of acting as adjuvants in parenteral vaccine formulations.

Thus, in a first aspect, the present invention relates to parenteral vaccine formulations comprising at least one immunogenic substance, and as an adjuvant titanium dioxide.

The advantages of the adjuvant salts are numerous. It has surprisingly been found that these salts act as adjuvants when included in parenteral vaccine formulations, implying that it will in many cases be possible to reduce the amount of immunogenic substance needed to induce an immune response. Moreover, the adjuvanicity of these salts can in some cases induce an immune response with an earlier onset and/or prolonged persistence, when compared to a conventionally used adjuvant. Accordingly, the number of booster vaccinations to obtain and maintain the desired level of immunity may be reduced. Moreover, studies indicate that the use of the salts described herein in vaccine formulations for parenteral use will in some cases increase the magnitude of the immune response, even to greater extents than seen with the conventionally used aluminium hydroxide.

A reason for this could be that the adjuvant salts exert their effect as adjuvants by creating a depot of the immunogenic substance, resulting in a slow and prolonged release of the immunogenic substance over time. On the other hand, the salts could also promote uptake of the immunogenic substance by antigen presenting cells. It should, however, be emphasised that this is a hypothesis, and should therefore have no limiting effect.

The salts can be characterised by a number of physical-chemical properties. These properties are believed to influence the salt's ability to act as an adjuvant to a greater or lesser extent. For instance, the solubility constant, the lattice energy, the nature of binding (covalent binding contra ionic binding), the pH, the oxidation stage, the particle size, and the ability to adsorb the immunogenic substance could all be involved. The compounds listed in the Examples have a low solubility and high lattice energy. Furthermore, the vaccine formulations of the Examples are slightly alkaline. Thus, some of the characteristics may be important in connecting with adjuvanicity. However, it is to be understood that the above are hypothesises and therefore should have no limiting effect on the scope of the invention.

The salts falling within the definition above are known chemical compounds, and some are currently being applied in veterinary or human medicine as laxatives, antacids or in cosmetic applications, cf. the examples in the table below. None of the salts have previously been used in parenteral vaccine formulations as sole adjuvants.

| Name | Compound | Solubility¹ | Current use | Dose² |
|---|---|---|---|---|
| Magnesium hydroxide | Mg(OH)₂ | Insoluble | Antacid (h), cathartic laxative (v) | 0.5 |
| Magnesium carbonate hydroxide pentahydrate | (MgCO₃)₄ Mg(OH)₂ 5H₂O | Low / insoluble | Antacid (h), laxative (v) | 0.5 |
| Titanium oxide | TiO₂ | Insoluble | Topical protection (h) / cosmetic applications (h) | 1.0 |

| | | | | |
|---|---|---|---|---|
| ¹ Solubility in water, according to manufacturer ² Recommended maximum dose (g per kg bodyweight) h approved for use in humans v approved for veterinary use | | | | |

As used herein, the term "adjuvant" refers to an immunological adjuvant. By this is meant a compound that is able to enhance the immune system's response to an immunogenic substance. The term "immunogenic" refers to a substance or active ingredient which when administered to a subject, either alone or with an adjuvant, induces an immune response in the subject. The term "immune response" includes specific humoral, i.e. antibody, as well as cellular immune responses, the antibodies being serologic as well as secretory and pertaining to the subclasses IgM, IgD, IgG, IgA and IgE as well as all isotypes, allotypes, and subclasses thereof. The term is further intended to include other serum or tissue components. The cellular response includes Type-1 and Type-2 T-helper lymphocytes, cytotoxic T-cells as well as natural killer (NK) cells.

The concept of vaccination/immunisation is based on two fundamental characteristics of the immune system, namely specificity and memory. The first vaccination/- immunisation will initiate a response specifically directed to the antigen with which the subject was challenged. Furthermore, a population of memory B and T lymphocytes will be induced. Upon re-exposure to the antigen or the pathogen it is derived from, the immune system will be primed to respond much faster and much more vigorously, thus endowing the vaccinated/immunised individual with immunological protection against the pathogen.

It lies within the scope of the present invention to use one or more immunogenic substances in the vaccine formulation. A case where the vaccine comprises more than one immunogenic substance is the so-called combination vaccines.

Examples of immunogenic substances are antigens, allergens, allergoids, peptides, proteins, haptens, carbohydrates, peptide nucleic acids (PNAs, a sort of synthetic genetic mimic), and viral or bacterial material as well as analogues or derivatives thereof. In the present context, the term "analogues or derivatives" is intended to include modified forms of the immunogenic substance. The modification can be made by chemical modification or synthetic modification, e.g. by PEGylation (PEG = polyethylene glycol), biotinylation, deamination, maleination, substitution of one or more amino acids, by cross-linking, by glycosylation, or by other recombinant or synthetic technology. The term is also intended to include natural-occurring mutations, isoforms and retroinverse analogues.

In particular such immunogenic substances may be natural, recombinant or modified proteins or fragments thereof, antigens, allergens, allergoids, peptides, haptens conjugated on a suitable carrier like KLH (key hole limpet hemocyanin) or *Tetanus toxoid*, carbohydrates, optionally inactivated or attenuated bacteria or virus as well as components thereof, RNA, DNA, PNA, parasites or retroviruses, parasitic material, mycoplasma, or toxins, e.g. such derived from

Tetanus toxoid, Diphtheria toxoid, Cholera toxin A and B subunits, Rubella, Rhabdovirus (rabies), Myoxoviruses, Paramyoxyviruses like parainfluenza virus, mumps and measles, Picornaviruses like poliovirus, coxsackievirus, echovirus and rhinovirus, Reoviruses, Poxviruses like small pox virus, Vaccinia virus and cowpox virus, Papovaviruses like polyoma virus, papilloma virus and SV-40, Adenoviruses, EBV like mononucleosis virus, Parvoviruses like HPV B19, Herpes viruses like Herpes simplex virus, and Herpes zoster virus (Varicella virus), Cytomegalovirus (CMV), Arboviruses like yellow fever and Dengue fever, Retroviruses like HIV, Hepatitis viruses like Hepatitis A, Hepatitis B and Hepatitis C, Haemophilius influenzae type B, Mycobacterium like M. tuberculosis, M. bovis, M. africanum, M. microti, M. avium, M. intracellulare, M. kansasii, M. gordonae, M. paratuberculosis, and M. lepramurium, Borrelia spp. like B. burgdorferi, in particular B. burgdorferi sensu lato and B. burgdorferi sensu stricto, B. garinii, B. afzelii, B. duttoni and B. recurrentis, Bordetella pertussis (whooping cough), Salmonella spp. like S. typhimurium and S. typhi, Treponema spp. like T. pallidum, Leptospira spp., Campylobacter spp. like C. jejuni, Helicobacter spp. like H. pylori, Pseudomonas spp., Legionella spp., Neisseria spp. like N. gonorrhoea and N. menigitidis, Chlamydia spp. like C. trachomatis, C. pneumonia and C. psittae, Enterobacter spp., Klebsiella spp., Yersinia spp., Vibrio spp. like Vibrio cholerae, Gardnerella spp., Rickettsia spp., Clostridium spp. like C. difficile, C. botulinum and C. tetani, Lactobacillus spp., Listeria spp., and Mycoplasma spp. like M. pneumoniae M. hominis, Plasmodium falciparum, and Leishmania donovani,
moulds and fungi such as Cladosporium, Alternaria, Aspergillus, Basidiomycetes, Candida albicans, and Penicillinum,
allergoids such as glutaraldehyde or PEG modified allergen complexes.

Examples of immunogenic substances used in combination vaccines are immunogenic substances involved with Diphteria-Tetanus-Wooping cough-Polio, Measles-Parotitis-Rubella, and Hepatitis A and B.

Non-limiting examples of allergens to be used in the parenteral vaccine of the invention include inhalation allergens originating i.a. from trees, grasses, herbs, fungi, house dust mites, storage mites, cockroaches and animal hair, feathers, and dandruff. Important pollen allergens from trees, grasses and herbs are such originating from the taxonomic orders of *Fagales, Oleales* and *Pinales* including i.a. birch (*Betula*), alder *(Alnus),* hazel *(Corylus),* hornbeam *(Carpinus)* and olive *(Olea),* the order of *Poales* including i.a. grasses of the genera *Lolium, Phleum, Poa, Cynodon, Dactylis* and *Secale,* the orders of *Asterales* and *Urticales* including i.a. herbs of the genera *Ambrosia* and *Artemisia.* Important inhalation allergens from fungi are i.a. such originating from the genera *Alternaria* and *Cladosporium.* Other important inhalation allergens are those from house dust mites of the genus *Dermatophagoides,* storage mites from the genus *Lepidoglyphys destructor,* those from cockroaches and those from mammals such as cat, dog, horse, cow, and bird. Further, allergens to be used may be derived from venom allergens including such originating from stinging or biting insects such as those from the taxonomic order of Hymenoptera including bees, wasps, and ants.

It is to be understood that the term derived from includes the naturally-occurring substance as well as isoforms thereof. Furthermore, the substance may be prepared by means of recombinant or synthetic techniques. Specific allergen components are known to the person skilled in the art and include e.g. *Bet v* 1 *(B. verrucosa*, birch), *Aln g 1 (Alnus glutinosa,* alder), *Cor a* 1 *(Corylus avelana,* hazel) and *Car b* 1 (*Carpinus betulus,* hornbeam) of the *Fagales* order. Others are *Cry j* 1 *(Pinales), Amb a* 1 and 2, *Art v* 1 *(Asterales), Par j* 1 *(Urticales), Ole e* 1 (*Oleales*), *Ave e* 1, *Cyn d* 1, *Dac g* 1, *Fes p* 1, *Hol I* 1, Lol *p* 1 and 5, *Pas n* 1, *Phl p* 1 and *5, Poa p* 1, 2 and 5, *Sec c* 1 and 5, and *Sor h* 1 (various grass pollens), *Alt* a 1 and *Cla h* 1 (fungi), *Der f* 1 and 2, *Der p* 1 and 2 (house dust mites, *D*. *farinae* and *D*. *pteronyssinus,* respectively), *Lep d* 1, *Bla g* 1 and 2, *Per* a 1 (cockroaches, *Blatella germanica* and *Periplaneta american*, respectively), *Fel*. d 1 (cat), *Can f* 1 (dog), *Equ c* 1, 2 and 3 (horse), *Apis m* 1 and 2 (honeybee), *Ves g* 1, 2 and 5, *Pol a* 1, 2 and 5 (all wasps) and *Sol i* 1, 2, 3 and 4 (fire ant).

In some cases, the parenteral vaccine formulation according to the invention may further comprise an additional adjuvant. Such additional adjuvant is selected from conventionally used adjuvants. Examples of such additional adjuvants include saponins such as Quil A and Qs-21, oil in water emulsions such as MF59, MPL, PLG, PLGA, aluminium salts, calcium phosphate, water in oil emulsions such as IFA (Freund's incomplete adjuvant) and CFA (Freund's complete adjuvant), interleukins such as IL-1β, IL-2, IL-7, IL-12, and INFγ, Adju-Phos^{®}, glucan, antigen formulation, Cholera Holotoxin, liposomes, DDE, DHEA, DMPC, DMPG, DOC/Alum Complex, ISCOMs^{®}, muramyl dipeptide, monophosphoryl lipid A, muramyl tripeptide, and phospatidylethanolamine (see also "Vaccine Design. The Subunit and Adjuvant Approach", Chapter 7 (ref. 6)). In a preferred embodiment, the additional adjuvant is selected from saponins such as Quil A and Qs-21, MF59, MPL, PLG, PLGA, calcium phosphate, and aluminium salts.

Furthermore, the parenteral vaccine formulation of the invention may suitably comprise one or more pharmaceutically acceptable excipients and carriers. Examples of such are diluents, buffers, suspending agents, wetting agents, solubilising agents, pH-adjusting agents, dispersing agents, preserving agents, and/or colorants.

The vaccine formulation of the inventions is administered by a parenteral route. The parenteral route includes intravenous, intramuscular, intraarticular, subcutaneous, intradermal, epicutaneous/transdermal, and intra-peritoneal administration.

The cation of the adjuvant salt is preferably present in the vaccine in an amount of from about 0.0004 to about 120 M, such as from about 0.004 to about 12 M, preferably from about 0.008 to about 6 M.

The amount of the additional adjuvant depends on the adjuvant and the immunogenic substance in question, and will be the subject to optimisation. However, the person skilled in the art will readily know how to optimise the amount of such additional adjuvant having regard to the other constituents to be included in the formulation.

In a further embodiment, of the parenteral vaccine formulation, the adjuvant is a combination of magnesium hydroxide and titanium dioxide, or magnesium carbonate hydroxide pentahydrate and titanium dioxide. In a still further embodiment, the adjuvant is a combination of magnesium hydroxide, magnesium carbonate hydroxide pentahydrate, and titanium oxide. Such parenteral vaccine formulations may also suitable comprise an additional adjuvant. Such additional adjuvants are selected from those indicated above. However, the additional adjuvant is preferably selected from saponins such as Quil A and Qs-21, MF59, MPL, PLG, PLG A, calcium phosphate, and aluminium salts.

Furthermore, it may be possible to manufacture vaccine formulations having combined characteristics such as earlier onset, prolonged persistence, increased potency, Th₁, Th₂ and/or cytotoxic response depending on the choice of adjuvants or combination of adjuvants. Thus, it could be beneficial to use different adjuvant schemes depending on the type of vaccination. For instance, an adjuvant providing an early onset may be used for the initial vaccination, and an adjuvant providing enhanced potency may be used for boosters. For this purpose, a combination of the adjuvants described herein may be very suitable.

By including an additional adjuvant, it may be possible to prepare vaccine formulations with further specific or enhanced properties. For instance, addition of MPL may result in a formulation that predominantly induces a Th₁ type immune response, whereas addition of saponins may result in a formulation that induces a Th₁ type immune response as well as a cytotoxic response. Furthermore, a combination of one or more adjuvant salts in the parenteral vaccine formulations of the invention and one or more additional adjuvants may enable an advantageous combined adjuvant effect, possibly enabling the modulation of Th₁, Th₂ and/or cytotoxic response(s).

In a fourth aspect, the present invention relates to the use of titanium dioxide as an adjuvant in a vaccine formulation for parenteral administration. In a currently preferred embodiment, the salt to be used is selected from magnesium hydroxide, magnesium carbonate hydroxide pentahydrate, and titanium dioxide, or a combination of magnesium hydroxide and magnesium carbonate hydroxide pentahydrate, magnesium hydroxide and titanium dioxide, magnesium carbonate hydroxide pentahydrate and titanium dioxide, or magnesium hydroxide, magnesium carbonate hydroxide pentahydrate, and titanium dioxide.

In a still further aspect, the present invention relates to the use of a vaccine formulation of the invention for the manufacture of a medicament for generating an immune response in a subject, which methods comprise administering to the subject.

The present invention also enables the vaccination or treatment of a vertebrate including a human being comprising administering to the subject a vaccine formulation of the invention.

As described above, the parenteral vaccine compositions of the present invention comprise at least one immunogenic substance. Thus, the inclusion of two, three, four, five, six or more immunogenic substances are contemplated and believed to be advantageous in some cases. The amount of immunogenic substance(s) depends on the immunogenic substance or combination of immunogenic substances in question. However, it is contemplated that the amount of immunogenic substance required to induce an immune response can, in some cases, be reduced due to the beneficial effects of the adjuvant salts. Thus, the amount of each immunogenic substance will typically be in the range of from 0.0001 to 100000 µg/dose, such as from 0.01 to 10000 µg/dose, from 0.1 to 1000 µg/dose, or from 1 to 100 µg/dose.

The administration of the vaccine formulation of the invention may be as single doses or as several doses. In certain cases, administration only once may be sufficient. In general, several doses should be given with intervals of a day, a week, two weeks, a month, or several months, etc. For example, a single dose may be given once, or a dose may be given as a primer, followed by one or more booster vaccinations, or a continuous vaccination regime like up to four doses per week, followed by one month without vaccinations, followed by up to four doses per week (optionally with increasing amount of immunogen), etc. Optionally different adjuvants or combination of adjuvants may be used in the different vaccinations. These are all examples, and the optimal vaccination regime depends on the immunogenic substance in question and several other factors. The person skilled in the art will readily know how to optimise this.

The adjuvant compositions of the invention can be prepared by forming a suspension or gel of the adjuvant salts by adding liquid, optionally containing buffer, other salts, solvents or excipients, to a dry form of the salt, or, alternatively, adding liquid optionally containing buffer, other salts, excipients, to a pre-equilibrated pre-formed gel of the adjuvant salts. The adjuvant composition may then be formulated to a vaccine formulation with desired immunogenic substance(s) by mixing the adjuvant composition with the immunogenic substance(s), and, if necessary, leaving them to equilibrate before filling. The adjuvant composition and the immunogenic substance(s) may alternatively be mixed in a more concentrated form, thereby enabling later dilution.

Thus in a seventh aspect, the present invention relates to a process for preparing a parenteral vaccine formulation according to the invention, which process comprises adding liquid to a dry form of or a pre-formed gel of the titanium dioxide thereby obtaining an adjuvant composition, and mixing said adjuvant composition with one or more immunogenic substances and optionally pharmaceutically acceptable carriers and/or excipients, thereby obtaining the parenteral vaccine formulation.

In a still further aspect, the present invention relates to parenteral vaccine formulations obtainable by the process defined above.

Containers for mixing and storage of the adjuvant compositions and vaccine formulations of the invention may be made of glass or various polymeric materials. The containers chosen should not adsorb the product stored. The containers may suitably be ampoules or capped vials for mono- or multidosage.

The invention is further illustrated by the following non-limited examples.

### EXAMPLES

Below, the procedures and protocols applied in carrying out the experiments of Examples 1 and 2 are described in general.

### Immunogenic substance:

*Tetanus toxoid* (TT) containing 3.0 mg protein/ml, (obtained from Statens Serum Institut, DK-2300 Copenhagen S, Denmark). Vaccine formulations containing aluminium hydroxide, as an adjuvant, contained either 30 µg, 10 µg or 1 µg TT per dose (300 µg/ml, 100 µg/ml, and 10 µg/ml, respectively), and vaccine formulations of the invention containing magnesium hydroxide, magnesium carbonate hydroxide pentahydrate or titanium dioxide, as an adjuvant of the invention, and the adjuvant free vaccine formulation (termed "no adjuvant") all contained 1 µg TT per dose (10 µg/ml).

### Adjuvants:

The molar concentration stated below are for the final vaccine formulations:

Aluminium hydroxide, Al(OH)₃ 0.045-0.05 M Al³⁺ (1.25 mg Al³+ /ml). Prepared from Alhydrogel 1.3%^{®} (Superfos, DK-2950 Vedbæk, Denmark).

Magnesium hydroxide, Mg(OH)₂ 0.05 M Mg ²⁺. Prepared from Mg(OH)₂ gel(Reheis, USA).

Magnesium carbonate hydroxide pentahydrate, (MgCO₃)₄Mg(OH)₂5H₂O, 0.05 M Mg ²⁺. Prepared from Magnesium carbonate hydroxide pentahydrate (Sigma USA).

Titanium dioxide, TiO₂, 0.05 M Ti⁴⁺. Prepared from Titanium dioxide pigment (Kemira, Finland).

### Preparation of the vaccine formulations:

The vaccine formulations were prepared as follows:

TT was dissolved or diluted to a concentration 10 times that of the concentration in the final vaccine formulation. The adjuvant was dissolved or diluted to a concentration five times that of the concentration in the final vaccine formulation, with regard to the cation. 1 volume TT solution was slowly mixed with 2 volumes adjuvant, and left stirring over night at 4°C. The following day 7 volumes Coca 0.0 buffer (0.25% sodium hydrogen carbonate and 0.5% sodium chloride) was slowly added. The adjuvant free vaccine was prepared as above, with the modification that the adjuvant was substituted with Coca 0.0 buffer.

### Immunisations :

For each vaccine formulation, groups of 8 female BALB/Ca mice, 6-8 weeks of age, were given subcutaneous immunisations, inguinally, on days 0 and 14. Each immunisation consisted of 100 µl vaccine.

Blood samples were drawn from the retro orbital vein every 7 days, starting on day 0. Serum was separated from the blood sample, and stored at -20°C, until analysed.

### Analysing serum samples:

Serum samples were analysed for the presence of TT-specific immune response by means of a direct enzyme linked immunosorbent assay (ELISA), measuring TT-specific antibodies of the IgG class. Briefly, immunosorbent plates (Nunc Maxisorp^{®}, Nunc, Denmark) were coated in a well known manner with TT, and free binding sites were blocked with bovine serum albumin. Serial dilutions of serum samples from the immunised mice were then added onto the plate, together with serial dilutions of a negative control serum pool from unimmunised BALB/Ca mice. A monoclonal TT-specific antibody (obtained from Statens Serum Institut, Copenhagen, Denmark), added in serial dilutions served both as a positive control, as well as an internal standard, used for determining the titre. All samples were added in duplicate.

Bound antibodies were detected by serial incubations of the plate with a biotinylated, polyclonal antiserum to mouse IgG (obtained from Jackson Laboratories, Bar Harbour, ME, USA) followed by a streptavidine-horseradish peroxidase conjugate (obtained from DAKO A/S, Glostrup, Denmark). The plates were developed for 20 minutes with 100 µl readymade TMB substrate (Kem-En-Tech, Denmark) per well, and the reaction was stopped with an equal volume of 1M H₂SO₄.

The developed colour reaction was measured as absorption at 450 nm.

### Data analysis:

The absorption at 450 nm was plotted against the dilution of the serum, for each individual mouse, as well as the geometric mean for individual groups.

The strength of the TT-specific antibody response in each serum sample was measured as an arbitrary titre, determined as the serum dilution giving an absorption signal equal to 50% of that obtained with a 3000 fold dilution of the TT-specific monoclonal antibody. Mice that did not respond, i.e., whose responses were indistinguishable from those seen with the serum pool from unimmunised control mice, were assigned a titre of 10. Mice that responded, but with responses below the magnitude required to determine a titre, were assigned a titre of 100.

### EXAMPLE 1

One immunisation with vaccine formulations of the invention comprising either magnesium hydroxide, magnesium carbonate hydroxide pentahydrate, or titanium dioxide as an adjuvant

The vaccines were prepared as described above, and the mice were immunised once on day 0 as described above. Blood was drawn on day 7, and serum was prepared and analysed as described above.

In Figure 1, the results are depicted as titres for individual mice, as well as the mean titre for each group. The adjuvant given is indicated below each group. The number in parenthesis indicates the amount (in µg) of immunogen (TT) given.

As can be seen from Figure 1, vaccine formulations comprising either magnesium hydroxide, magnesium carbonate hydroxide pentahydrate (in short MgCO₃), or titanium dioxide as an adjuvant, were more potent at inducing TT-specific antibodies, than vaccine formulation containing Al(OH)₃ as an adjuvant. Induction of TT-specific antibodies using an Al(OH)₃-containing vaccine formulations required 10-30 times as much immunogen (TT) as vaccine formulations comprising either magnesium hydroxide, magnesium carbonate hydroxide pentahydrate, or titanium dioxide as an adjuvant. Thus, the experiment clearly shows that when the adjuvants of the invention is included in parenteral vaccine formulations, the amount of antigen necessary to induce an immune response, following one immunisation, is reduced.

### EXAMPLE 2

Immunisations with vaccine formulations of the invention comprising either magnesium hydroxide, magnesium carbonate hydroxide pentahydrate, or titanium dioxide as an adjuvant

The vaccine formulations were prepared as described above, and the mice were immunised on day 0 and 14 with 1 µg of TT as described above. Blood was drawn every 7 days from day 0 to 42, and serum was prepared and analysed as described above.

Results, given as the geometric mean titre of the whole group, are shown for days 7 and 28 in Figure 2A and 2B. The adjuvant used is indicated below each group. As can be seen in Figure 2A, vaccine formulations comprising either magnesium hydroxide, magnesium carbonate hydroxide pentahydrate (in short MgCO₃), or titanium dioxide as an adjuvant induce specific immune responses with an earlier onset than vaccine formulations containing Al(OH)₃ as an adjuvant. Furthermore, as can be seen in Figure 2B, following a second vaccination of all mice on day 14, the immune responses induced with vaccine formulations containing either magnesium hydroxide, magnesium carbonate hydroxide pentahydrate (in short MgCO₃), or titanium dioxide as an adjuvant, induce specific immune responses similar in magnitude to those induced by a vaccine containing Al(OH)₃ as an adjuvant.

Thus, when the adjuvant salts are included in parenteral vaccine formulations, a persistent, specific immune response is induced. Furthermore, an earlier onset is observed, and the magnitude of the immune response is comparable to that seen with vaccine formulations containing Al(OH)₃ as an adjuvant.

### REFERENCES

1. R.K. Gupta, B.E. Rost, E. Relyveld, and G.R. Siber, Adjuvant properties of aluminium and calcium compounds. M.F. Powell and M.J. Newman (Eds.), Vaccine Design. The Subunit and Adjuvant Approach. 1995 Plenum Press, New York, N.Y., pages 229-248
2. J.K. Rao, C.D. Katsetos, M.M. Herman and J. Savory, Experimental aluminium encephalomyelopathy. Relationship to human neurodegenerative disease. Clin. Lab. Med. 18(4), 687-698 viii (December 1998)
3. J. Savory, C. Exley, W.F. Forbes, Y. Huang, J.G. Joshi, T. Kruck. D.R. McLachlan, and I. Wakayama, J. Toxicol. Environ. Health 48(6), 615-635 (30 August 1996)
4. M.J. Newman, Vaccine adjuvants, Exp. Opin. Ther. Patents 10(3), 1-18 (2000)
5. D.T. O' Hagan, G.S. Ott, and G. Van Nest, Recent advances in vaccine adjuvants: the development of MF59 emulsion and polymeric microparticles, Mol. Med. Today 3(2), 69-75 (February 1997)
6. "Vaccine Design. The Subunit and Adjuvant Approach", Chapter 7
7. J.Y. Lee et al.: "Beryllium, an adjuvant that promotes gamma interferon production", Infection and Immunity, Vol. 68, No. 7, 4032-4039 XP002169720 (July 2000)
8. Stas' N.F. et al.: "Modification of Aluminium Hydroxide used as an adjuvant", Database Biosis, Khimiko-Farmatsevticheskii Zhurnal, Vol. 24, No. 7, 65-66 (1990)
9. Singh m; o'Hagan d: "advances in vaccine adjuvants" nature biotechnology, nature publishing group, new york, ny, us, vol. 17, no. 11, 1 november 1999 (1999-11-01), pages 1075-1081, xp000999107 issn: 1087-0156
10. Costagliola et al.: "Recombinant thyrotropin receptor and the induction of autoimmune thyroid disease in balb/c mice: A new animal model." Endocrinology, vol. 135, no. 5, 1994, pages 2150-2159, xp001009706 philadelphia, US

## Claims

1. A parenteral vaccine formulation comprising at least one immunogenic substance, and as an adjuvant titanium dioxide.

2. A parenteral vaccine formulation according to claim 1 further comprising an additional adjuvant.

3. A parenteral vaccine formulation according to claim 2, wherein the additional adjuvant is selected from saponins such as Quil A and Qs-21, MF59, MPL, PLG, PLGA, calcium phosphate, and aluminium salts.

4. A parenteral vaccine formulation according to any of claims 1-3 further comprising pharmaceutically acceptable excipients and/or carriers.

5. A parenteral vaccine formulation according to any of claims 1-4 further comprising diluents, buffers, suspending agents, solubilising agents, pH-adjusting agents, dispersing agents, and/or colorants.

6. A parenteral vaccine formulation according to any of claims 1-5 for intravenous, intramuscular, intraarticular, subcutaneous, intradermal, epicutaneous, and intraperitoneal administration.

7. A parenteral vaccine formulation according to any of claims 1-6, wherein the cation of the adjuvant is present in an amount of from about 0.0004 to about 120 M, such as from about 0.004 to about 12 M, or in an amount of from about 0.008 to about 6 M.

8. A parenteral vaccine formulation according to any of claims 1-7, wherein the adjuvant is a combination of magnesium carbonate hydroxide pentahydrate and titanium dioxide, magnesium hydroxide and titanium dioxide, or magnesium hydroxide, magnesium carbonate hydroxide pentahydrate and titanium dioxide.

9. Use of titanium dioxide as an adjuvant for the manufacture of a vaccine formulation for parenteral administration.

10. Use according to claim 9 wherein magnesium hydroxide is combined with titanium dioxide, titanium dioxide is combined with magnesium carbonate hydroxide pentahydrate, or magnesium hydroxide, magnesium carbonate hydroxide pentahydrate, and titanium dioxide is combined.

11. Use according to claims 9 or 10 wherein the vaccine formulation contains at least one immunogenic substance.

12. Use according to claim 11 wherein the immunogenic substance is an allergen, or an allergoid.

13. Use of a vaccine formulation according to any of claims 1-8 for the manufacture of a medicament for generating an immune response in a subject.

14. A process for preparing a parenteral vaccine formulation according to any of claims 1-8 comprising adding liquid to a dry form or a pre-formed gel of the titanium dioxide, thereby obtaining an adjuvant composition, and mixing said adjuvant composition with one or more immunogenic substances and optionally pharmaceutically acceptable carriers and/or excipients, thereby obtaining the parenteral vaccine formulation.

15. Parenteral vaccine formulation according to any one of claims 1-8 comprising a gel or suspension of the adjuvant salt(s) mixed with immunogenic substance(s).

## Patentansprüche

1. Parenterale Impfstoff-Formulierung umfassend mindestens eine immunogene Substanz und Titandioxid als Hilfsstoff.

2. Parenterale Impfstoff-Formulierung nach Anspruch 1, welche ferner einen zusätzlichen Hilfsstoff umfasst.

3. Parenterale Impfstoff-Formulierung nach Anspruch 2, wobei der zusätzliche Hilfsstoff aus Saponinen, wie beispielsweise Quil A und Qs-21, MF59, MPL, PLG, PLGA, Calciumphosphat und Aluminiumsalzen, ausgewählt ist.

4. Parenterale Impfstoff-Formulierung nach einem der Ansprüche 1 bis 3, welche ferner pharmazeutisch akzeptable Bindemittel und/oder Träger umfasst.

5. Parenterale Impfstoff-Formulierung nach einem der Ansprüche 1 bis 4, welche ferner Verdünnungsmittel, Puffer, Suspensionsmittel, Solubilisierungsmittel, Säureregulatoren, Dispergiermittel und/oder Farbmittel umfasst.

6. Parenterale Impfstoff-Formulierung nach einem der Ansprüche 1 bis 5 zur intravenösen, intramuskulären, intraartikulären, subkutanen, intradermalen, epikutanen und interperitonealen Verabreichung.

7. Parenterale Impfstoff-Formulierung nach einem der Ansprüche 1 bis 6, wobei die Kation des Hilfsstoffes in einer Menge im Bereich von etwa 0,0004 bis etwa 120 M, wie beispielsweise im Bereich von etwa 0,004 bis etwa 12 M, oder in einer Menge im Bereich von etwa 0,008 bis etwa 6 M vorliegt.

8. Parenterale Impfstoff-Formulierung nach einem der Ansprüche 1 bis 7, wobei der Hilfsstoff eine Kombination aus Magnesiumcarbonathydroxidpentahydrat und Titandioxid, Magnesiumhydroxid und Titandioxid oder Magnesiumhydroxid, Magnesiumcarbonathydroxidpentahydrat und Titandioxid darstellt.

9. Verwendung von Titandioxid als Hilfsstoff für die Herstellung einer Impfstoff-Formulierung zur parenteralen Verabreichung.

10. Verwendung nach Anspruch 9, wobei Magnesiumhydroxid mit Titandioxid kombiniert wird, Titandioxid mit Magnesiumcarbonathydroxidpentahydrat kombiniert wird, oder Magnesiumhydroxid, Magnesiumcarbonathydroxidpentahydrat und Titandioxid kombiniert werden.

11. Verwendung nach den Ansprüchen 9 oder 10, wobei die Impfstoff-Formulierung mindestens eine immunogene Substanz enthält.

12. Verwendung nach Anspruch 11, wobei die immunogene Substanz ein Allergen oder ein Allergoid ist.

13. Verwendung einer Impfstoff-Formulierung nach einem der Ansprüche 1 bis 8 zur Herstellung eines Medikamentes zur Erzeugung einer Immunantwort in einem Individuum.

14. Verfahren zur Herstellung einer parenteralen Impfstoff-Formulierung nach einem der Ansprüche 1 bis 8 umfassend die Zugabe von Flüssigkeit zu einem trockenförmigen oder vorgeformten Gel des Titandioxids, wodurch eine Hilfsstoffzusammensetzung erhalten wird, und Vermischen der Hilfsstoffzusammensetzung mit einer oder mehreren immunogen Substanzen und wahlweise mit pharmazeutisch akzeptablen Trägern und/oder Bindemitteln, wodurch die parenterale Impfstoff-Formulierung erhalten wird.

15. Parenterale Impfstoff-Formulierung nach einem der Ansprüche 1 bis 8 umfassend ein Gel oder eine mit immunogener Substanz oder immunogenen Substanzen gemischte Suspension des Salzes oder der Salze des Hilfsstoffes.

## Revendications

1. Formulation parentérale de vaccin comprenant au moins une substance immunogène, et en tant qu'un adjuvant le dioxyde de titane.

2. Formulation parentérale de vaccin selon la revendication 1, comprenant en outre un adjuvant supplémentaire.

3. Formulation parentérale de vaccin selon la revendication 2, dans laquelle l'adjuvant supplémentaire est choisi parmi les saponines telles que Quil A et Qs-21, MF59, MPL, PLG, PLGA, le phosphate de calcium et des sels d'aluminium.

4. Formulation parentérale de vaccin selon l'une quelconque des revendications 1 à 3, comprenant en outre des excipients et/ou des transporteurs pharmaceutiquement acceptables.

5. Formulation parentérale de vaccin selon l'une quelconque des revendications 1 à 4, comprenant en outre des diluants, des tampons, des agents de suspension, des agents solubilisants, des agents d'ajustement de la valeur pH, des agents dispersants et/ou des colorants.

6. Formulation parentérale de vaccin selon l'une quelconque des revendications 1 à 5 pour l'administration intraveineuse, intramusculaire, intra-articulaire, sous-cutanée, intradermique, epicutanée et intrapéritonéale.

7. Formulation parentérale de vaccin selon l'une quelconque des revendications 1 à 6, dans laquelle le cation de l'adjuvant est présent dans une quantité d'environ 0,0004 à environ 120 M, par exemple d'environ 0,004 à environ 12 M, ou dans une quantité d'environ 0,008 à environ 6 M.

8. Formulation parentérale de vaccin selon l'une quelconque des revendications 1 à 7, dans laquelle l'adjuvant est une combinaison de l'hydroxyde de carbonate de magnésium pentahydraté et du dioxyde de titane, de l'hydroxyde de magnésium et du dioxyde de titane, ou de l'hydroxyde de magnésium, de l'hydroxyde de carbonate de magnésium pentahydraté et du dioxyde de titane.

9. Utilisation du dioxyde de titane en tant qu'adjuvant pour la fabrication d'une formulation de vaccin pour l'administration parentérale.

10. Utilisation selon la revendication 9, dans laquelle l'hydroxyde de magnésium est combiné avec le dioxyde de titane, le dioxyde de titane est combiné avec de l'hydroxyde de carbonate de magnésium pentahydraté, ou de l'hydroxyde de magnésium, l'hydroxyde de carbonate de magnésium pentahydraté, et le dioxyde de titane est combiné.

11. Utilisation selon les revendications 9 ou 10, dans laquelle la formulation de vaccin contient au moins une substance immunogène.

12. Utilisation selon la revendication 11, dans laquelle la substance immunogène est un allergène, ou un allergoïde.

13. Utilisation d'une formulation de vaccin selon l'une quelconque des revendications 1 à 8 pour la fabrication d'un médicament destiné à générer une réponse immunitaire chez un sujet.

14. Procédé de préparation d'une formulation parentérale de vaccin selon l'une quelconque des revendications 1 à 8, comprenant l'addition de liquide à une forme sèche ou à un gel préformé du dioxyde de titane pour obtenir ainsi une composition d'adjuvant, et le mélange de ladite composition d'adjuvant avec une ou plusieurs substances immunogènes et éventuellement les transporteurs et/ou les excipients pharmaceutiquement acceptables, pour obtenir ainsi la formulation parentérale de vaccin.

15. Formulation parentérale de vaccin selon l'une quelconque des revendications 1 à 8, comprenant un gel ou une suspension du sel adjuvant ou des sels adjuvants mélangé(s) avec la substance immunogène ou les substances immunogènes.
